# EUROPEAN PATENT APPLICATION

(11) **EP 1 574 227 A1**
(43) Date of publication of application: **14.09.2005**
(21) Application number: 04005536.0
(22) Date of filing: 09.03.2004
(51) Int. Cl.: A61L 15/58, A61L 15/42

(54) **Disposable absorbent articles with improved fastening performance to hydrophobic materials, particularly microfibre materials**

(71) Applicant: THE PROCTER & GAMBLE COMPANY, Cincinnati, Ohio 45202 (US)
(72) Inventor: Rosati, Rodrigo, 66023 Francavilla al Mare (CH) (IT); Gagliardi, Ivano, 65123 Pescara (IT); Veglio, Paolo, 65129 Pescara (IT); Carlucci, Giovanni, 66100 Chieti (IT)
(74) Representative: Kremer, Véronique Marie Joséphine

(57) **Abstract**

The present invention relates to an absorbent article for personal hygiene, especially a sanitary napkin, panty liner and the like, comprising an adhesive for securing said article to the garments of a wearer, especially microfibre garments. Said adhesive has an elastic modulus G' at 0.01 Hz and 25°C of from 1,000 to 10,000 Pa and a loss tangent tanδ at 0.01 Hz and 25°C of from 0.3 to 2.

## Description

### Field of Invention

The present invention relates to an absorbent article for personal hygiene, especially a sanitary napkin, panty liner and the like, comprising an adhesive for securing said article to the garments of a wearer, especially microfibre garments. Said adhesive has an elastic modulus G' at 0.01 Hz and 25°C of from 1,000 to 10,000 Pa and a loss tangent tanδ at 0.01 Hz and 25°C of from 0.3 to 2.

### Background of the Invention

The use of adhesives for securing disposable absorbent articles for personal hygiene is well known in the art. In particular, the use of hot melt and emulsion-based adhesives is general technical standard. The application of emulsion-based adhesives onto the backsheets of absorbent articles for garment fastening is for instance known from SE-A-374,489. The use of hot melt adhesives for this purpose is for instance described in EP-A-140,135 or in WO 00/61054.

In the recent time a significant change with respect to the clothing habits of women could be noticed. An increasing share of especially younger women does not wear cotton panties anymore, which were the standard of the last decades, but more and more tends to wear panties consisting of a particular synthetic fabric material, which is commonly referred to as "micro-fibre".

Micro fibres are one of the recent major developments in the fabric industry. These fibres conventionally have less than 1 denier and a diameter in cross section of conventionally not more than 10 µm. Soon after their occurrence on the market micro fibres have found use in especially the clothing industry, where they are used to form fabrics having unique physical and mechanical performance, such as luxurious look and feel due to the fact that microfibres are even thinner than silk, together with very good strength, uniformity and processing characteristics. The resulting very fine and close woven and knitted fabrics are characterized by soft handle and breathability. Due to this, microfibre fabrics are also used for the production of underpants for women, especially fashionable ones for younger women.

Microfibre fabrics have very different physical characteristics compared to conventional cotton fabrics. This especially applies to hydrophobicity, which is higher for microfibres and is oftentimes even increased by the treatment of the microfibres with fluoropolymers, silicones, microwaxes and the like. Thus, unlike conventional cotton garments, microfibre garments are provided with a substantially hydrophobic surface. Furthermore, the density of the fabrics made of microfibres is significantly higher compared to those made of cotton. As a consequence, the void space between the individual microfibre filaments is much lower compared to the void space in cotton fabrics. Because of the aforementioned characteristics currently available conventional adhesives for fastening absorbent articles, such as sanitary napkins and panty liners, to garments do not work satisfactory for microfibre garments. It has been observed that the bonding forces the current panty fastening adhesives (hereinafter PFAs) are able to deliver on microfibre garments are by far too low for reliable attachment of absorbent articles, especially under stress conditions, such as for instance during physical exercise and the like.

It is therefore an object of the present invention to provide an absorbent article with a PFA, which is capable to provide secure attachment of the absorbent article on microfibre-based garments.

### Summary of the Invention

It has been found that the viscoelastic behaviour of the adhesive, which is described by the rheologic parameters tanδ, G' and G" is the key element to be fine-tuned in order to solve the above stated problem.

The present invention provides an absorbent article being provided with a PFA for attachment to the garments of a wearer. In particular, the absorbent article of the present invention is provided with a PFA having an elastic modulus G' at 0.01 Hz and 25°C of from 1,000 to 10,000 Pa and a loss tangent tanδ at 0.01 Hz and 25°C of from 0.3 to 2.

In a preferred embodiment of the present invention provides an absorbent article being provided with a PFA having a tanδ residing inside a quadrangle ABCD, wherein said quadrangle ABCD is defined by graphically plotting frequency in Hz versus tanδ referenced to 25°C of said adhesive, said quadrangle ABCD having as points A and D a tanδ of 0.3 and 2, respectively, at a frequency of 0.01 Hz and points B and C at a tanδ of 0.8 and 4, respectively, at a frequency of 10 Hz.

In another preferred embodiment of the present invention an absorbent article is provided with a PFA having a G' residing inside a quadrangle ABCD wherein said quadrangle ABCD is defined by graphically plotting frequency in Hz versus tanδ referenced to 25°C of said adhesive, said quadrangle ABCD having as points A and D a G' of 1,000 and 10,000, respectively, at a frequency of 0.01 Hz and points B and C at a G' of 20,000 and 100,000, respectively, at a frequency of 10 Hz.

### Brief Description of the Drawings

Figure 1 is a graphic representation of the parameter ranges claimed in claim 1, namely of tanδ and G' at 0.01 Hz and 25°C.
Figure 2 is a double-logarithmic graphic representation of the parameter ranges claimed in claim 4, namely of tanδ in a frequency interval of from 0.01 Hz to 10 Hz at 25°C.
Figure 3 is a double-logarithmic graphic representation of the parameter ranges claimed in claim 6, namely of G' in a frequency interval of from 0.01 Hz to 10 Hz at 25°C.
Figure 4 is a graphic representation of the test results for the four exemplary PFAs investigated herein with respect to the parameters claimed in claim 1.
Figure 5 is a graphic representation of the test results for the four exemplary PFAs investigated herein with respect to the parameters claimed in claim 4.
Figure 6 is a graphic representation of the test results for the four exemplary PFAs investigated herein with respect to the parameters claimed in claim 6.
Figure 7 illustrates the tape side of the standard microfibre material used in the peel force test method herein.
Figure 8 illustrates the PFA side of the standard microfibre material used in the peel force test method herein.

### Detailed Description of the Invention

The term 'absorbent article' is used herein in a very broad sense including any article being able to receive and/or absorb and/or contain and/or retain fluids and/or exudates, especially bodily fluids/bodily exudates. The absorbent article, which is referred to in the present invention typically comprises a fluid pervious topsheet as the wearer-facing layer, a fluid impervious backsheet as the garment-facing layer that is preferably water vapour and/or gas pervious and an absorbent core comprised there between. Furthermore, absorbent articles in the context of the present invention are provided with a means for their attachment to the user's garment, in particular with an adhesive. Preferred absorbent articles in the context of the present invention are disposable absorbent articles. Typical disposable absorbent articles according to the present invention are absorbent articles for personal hygiene, such as baby care articles like baby diapers; incontinence pads and perspiration pads like underarm sweat pads or hat bands. Particularly preferred disposable absorbent articles are absorbent articles for feminine hygiene like sanitary napkins and panty liners.

By 'body fluid' it is meant herein any fluid produced by the human body including for instance perspiration, urine, blood, menstrual fluids, vaginal secretions and the like.

The term 'disposable' is used herein to describe articles, which are not intended to be laundered or otherwise restored or reused as an article (i.e. they are intended to be discarded after a single use and preferably to be recycled, composted or otherwise disposed of in an environmentally compatible manner).

The term 'use', as used herein, refers to the period of time that starts when the absorbent article is actually put in contact with the anatomy of the user.

As used herein, 'hydrophilic' refers to a material having a contact angle of water in air of less than 90 degrees, whereas the term 'hydrophobic' herein refers to a material having a contact angle of water in air of 90 degrees or greater. Hydrophobic materials are also referred to as water-repellent. 'Microfibres' as referred to herein means fibres having a denier of not more than 1 (1 denier=1 g / 9000 m of fibre) and a diameter in cross section of not more than 10 µm. Microfibres are artificial man-made fibres and most typically consist of polyester or polyamides, such as nylon. Microfibres are used by the fabric industry for making very fine close woven fabric materials and knitted fabrics, which are characterized by soft handle and breathability. Microfibre fabrics have very different physical characteristics compared to conventional cotton fabrics. This especially applies for hydrophobicity, which is significantly higher than the one of cotton fibres and which oftentimes is even increased by the treatment of the microfibres with fluoropolymers, silicones, microwaxes and the like. Oftentimes microfibre garments also contain elastan/Lycra fibres for providing elasticity. Due to the small diameter of the microfibres the density of the fabrics made therefrom is very high compared to the one of cotton fabrics. Thanks to the small fibre diameter of those microfibres the void space between the individual microfibres is very low compared to cotton fabrics. Typical microfibre materials are marketed by e.g. DuPont under the trade name Tactel® or by Nylstar under the trade name Meryl®.

As said *infra,* an absorbent article in the context of the present invention comprises an adhesive means for the attachment of this article to the user's garments. This adhesive is also referred to as 'panty fastening adhesive' or 'PFA'. The PFA is provided on the garment facing surface of the absorbent article of the present invention for attaching said article to the garment of a wearer. Similarly, if the product is a winged product, the wings can also be provided with PFA on the garment-facing surface in order to secure the wings to the wearer's garment. The PFA for use herein is preferably a pressure-sensitive adhesive, with hot melt pressure-sensitive adhesives being particularly preferred.

The present inventors have found that the key property of an adhesive for being able to reliably bond to a microfibre garment is its viscoelastic behaviour. In fact it is well known from literature (see e.g. "Viscoelastic windows of pressure sensitive adhesives", E. P. Chang, J. Adhesion, Vol.34, 1991, pp. 189-200; Handbook of pressure sensitive adhesive technology, Ed. Satas, 3rd edition, pp. 171-183; Pocius "Adhesion and adhesive technology", pp. 216-245) that the performance of adhesives, e.g. peel, tack, shear, depends strongly on the bulk viscoelastic properties of the adhesives. This viscoelastic behaviour can be quantified by the rheological parameters elastic modulus G', viscous modulus G" and loss tangent tanδ (which is the ratio G"/G'). All these parameters are specific for certain frequencies and can be determined by the standard test procedure ASTM D4440-95, using flat plates oscillating at the frequency of interest.

Firstly it has to be noted that the rheological parameters greatly vary with frequency. Secondly, the frequency in the measurement procedure for the rheological parameters simulates certain real-life situations. Specifically, a frequency of 0.01 Hz represents a typical bonding and particularly a typical wearing situation, this means, that the behaviour of the adhesive upon attachment to the wearer's garment and particularly during wearing, after the attachment. Debonding of an article, specifically detachment from the wearer's garment is happening at higher frequencies. It has been found that 10 Hz is a suitable frequency to observe the adhesive's behaviour upon debonding.

The elastic and viscous moduli are determined herein at a temperature of 25°C (77° Fahrenheit). The adhesive used as PFA in the present invention must satisfy the following condition:

The loss tangent tanδ at 0.01 Hz and 25°C must be in the range of from 0.3 to 2 and the elastic modulus G' at 0.01 Hz and 25°C must be in the range of from 1,000 to 10,000 Pa. In fact it is critical for the attachment onto microfibre fabric that the adhesive can wet the microfibre surface and continue to wet the surface over the wearing time. Therefore we consider rheology properties on longer times, i.e. smaller frequencies, 0.01Hz (i.e. 0.0628rad/sec), very close to the creep frequency. Generally adhesives not satisfying the two conditions in this paragraph will provide insufficient adhesion to the microfibre fabric surface.

It has been found that if the loss tangent tanδ at 0.01Hz and 25°C is lower than 0.4 the adhesive will not be able to flow during the wearing time penetrating within the fiber net of the microfibre material, thus providing mechanical gripping and increasing the bonding force; the inventors have found that a tanδ at 0.01Hz and 25°C up to 2 is able to guarantee sufficient adhesion to microfibre fabric surface.

It has been found that if the elastic modulus G' at 0.01Hz and 25°C is higher than 10000 Pa, the adhesive is too hard so it will not be able to form a good bonding to the microfibre surface: in fact, during the wearing time, after that the adhesive has been applied under pressure and that the pressure has been released, the high elastic modulus enables the adhesive to have an elastic relaxation winning the low work of adhesion to the very hydrophobic microfibre material, thus unwetting the microfibre fibers, resulting in very low peel adhesion. The inventors have found that an elastic modulus G' at 0.01Hz and 25°C higher than 1000 Pa is able to guarantee sufficient adhesion to microfibre fabric surface.

Adhesive compositions, which satisfy the above criteria, can be used as PFA for the absorbent article of the present invention provided they also satisfy the common requirements of being safe for use in close proximity of human skin during use and generally after disposal of the article.

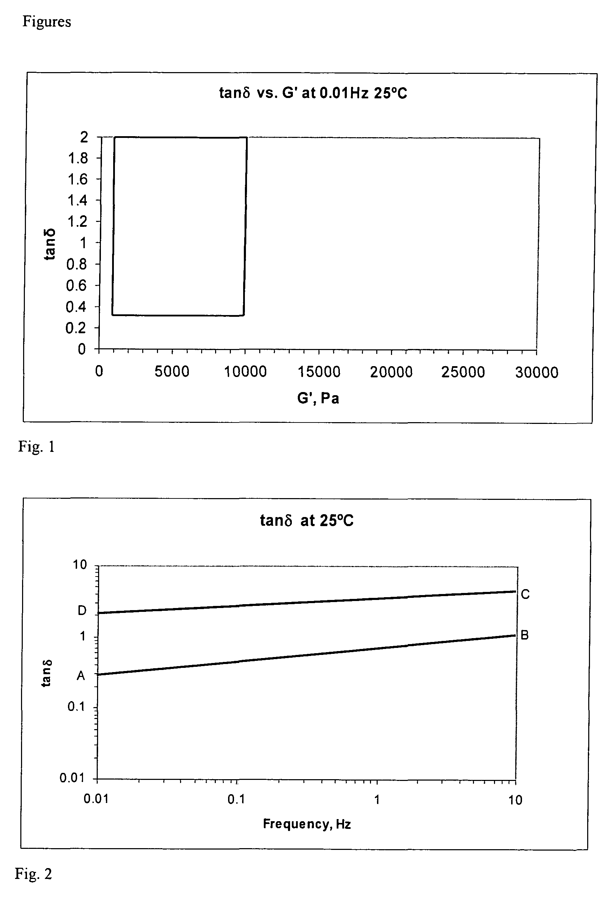

In a preferred embodiment the adhesive to be used as PFA according to the present invention has a tanδ residing inside a quadrangle ABCD, wherein said quadrangle ABCD is defined by graphically plotting tanδ versus frequency in Hz referenced to 25°C of said adhesive, said quadrangle ABCD having as points A and D a tanδ of 0.3 and 2, respectively, at a frequency of 0.01 Hz and points B and C at a tanδ of 0.8 and 4, respectively, at a frequency of 10 Hz. If tanδ is below the limits AB of the window described in this paragraph the adhesive is less flowable and thus its ability to penetrate into the interfibre spaces between the microfibres of the garment is reduced. The inventors have found that a tanδ up to the limit CD of Figure 2 is able to guarantee sufficient adhesion to microfibre fabric surface.

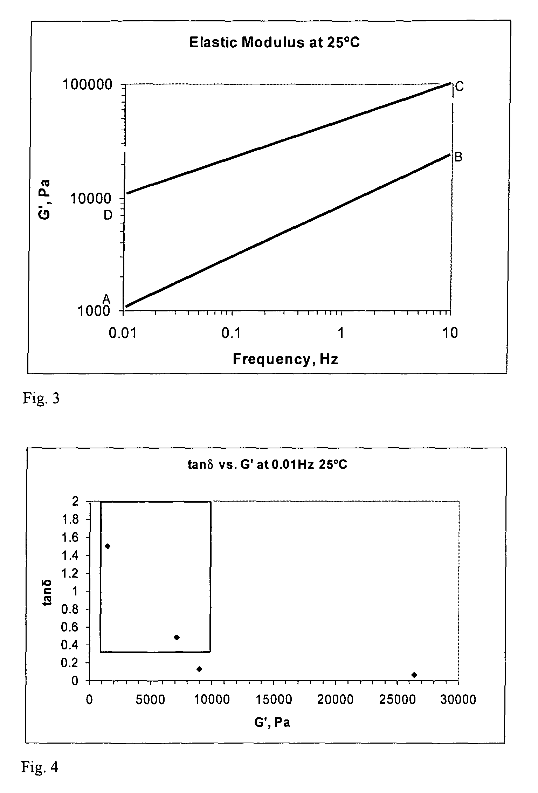

In another preferred embodiment the adhesive to be used as PFA according to the present invention has G' residing inside a quadrangle ABCD wherein said quadrangle ABCD is defined by graphically plotting G' versus frequency in Hz referenced to 25°C of said adhesive, said quadrangle ABCD having as points A and D a G' of 1,000 and 10,000, respectively, at a frequency of 0.01 Hz and points B and C at a G' of 20,000 and 100,000, respectively, at a frequency of 10 Hz. If G' is above the window CD defined in this paragraph the adhesive has reduced bonding properties because it is too rigid, in other words too hard. The inventors have found that an elastic modulus G' higher than the limit AB of figure 3 is able to guarantee sufficient adhesion to microfibre fabric surface.

In a particularly preferred embodiment of the present invention the adhesive has both properties defined in the two preceding paragraphs.

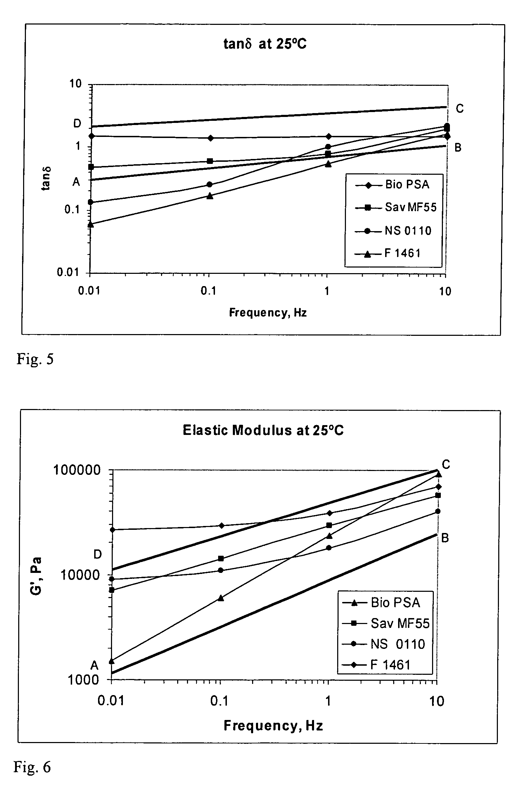

The plots in Figures 2, 3, 5 and 6 are presented on a double-logarithmic scale, as commonly done to describe rheological properties variations over several decades of frequency.

In the following, examples for adhesives suitable to be used as PFA for secure attachment of an absorbent article to a microfibre substrate are listed:

Commercially available examples for PFAs suitable herein are Bio PSA 7-4560 from Dow Corning, which is a silicone-based adhesive, and MF55 from Savare', which is an adhesive based on block-copolymerisates. In the following a detailed description of suitable silicone-based PFAs is given. As said above, Bio PSA 7-4560 from Dow Corning is part of this class of PFAs.

A suitable class of silicone-based pressure sensitive adhesive compositions is disclosed in US 4,865,920 and consists of (i) a trimethylsilyl-endblocked polysilicate resin such as a silicone resin consisting of a benzene-soluble resinous copolymer containing silicon-bonded hydroxyl radicals and consisting essentially of triorganosiloxy units of the formula R₃SiO_{1/2} and tetrafunctionalsiloxy units of the formula SiO_{4/2} in a ratio of about 0.6 to 0.9 triorganosiloxy units for each tetrafunctionalsiloxy unit present in the copolymer and (ii) a silanol-endstopped polydiorganosiloxane fluid (silicone fluid), e.g. a polydimethylsiloxane fluid. U.S. Patent No. 2,736,721 to Dexter et al. and U.S. Patent No. 2,814,601, to Currie et al. teach such or similar pressure sensitive adhesive compositions.

Another class of suitable pressure sensitive adhesive compositions to use with the specific ester disclosed herein *supra,* is that or those similar to that of U.S. Patent No. 2,857,356 to Goodwin, Jr. The Goodwin, Jr. patent teaches silicone pressure sensitive adhesive compositions which consist of a mixture of ingredients comprising (i) a cohydrolysis product of a trialkyl hydrolyzable silane and alkyl silicate, wherein the cohydrolysis product contains a plurality of silicon-bonded hydroxy groups (silicone resin), and (ii) a linear, high viscosity organopolysiloxane fluid (silicone fluid) containing silicon-bonded hydroxy groups.

The silicone resin (i) and the silicone fluid (ii) may optionally be condensed together such as by the procedure described in Canadian Patent No. 711,756 to Pail. In such a condensation reaction, the silicone resin (i) and the silicone fluid (ii) are mixed together in the presence of a silanol condensation catalyst and the silicone resin (i) and the silicone fluid (ii) are condensed, for example, by heating under reflux condition for 1 to 20 hours. Examples of silanol condensation catalysts are primary, secondary and tertiary amines, carboxylic acids of these amines and quaternary ammonium salts.

Another class of suitable pressure sensitive adhesive compositions to use with the specific ester disclosed herein *supra* are those compositions described in U.S. Patent Nos. 4,591,622 and 4,584,355 to Blizzard et al. U.S. Patent No. 4,585,836 to Homan et al. and U.S. Patent No. 4,655,767 to Woodard et al. Generally, these pressure sensitive adhesive compositions consist of a blend of a i) a silicone resin and ii) a silicone fluid which are chemically treated so as to reduce the silicon-bonded hydroxyl content of the blend. These compositions may optionally be condensed as described immediately above prior to the chemical treatment.

The silicone pressure sensitive adhesive is prepared by merely mixing siloxanes (i) and (ii) with the selected ester or esters. The silicone pressure sensitive adhesive compositions are then heated to a coatable viscosity and coated on the garment-facing surface of the absorbent article. Optionally, the coated silicone pressure sensitive adhesive may be cured. When the silicone pressure sensitive adhesive is to be cured, it may further contain a curing catalyst. It is preferred that such catalysts remain inactive at room temperature and temperatures reached during the hot-melt coating process. Therefore, such catalysts that either become active at temperatures higher than that of the hot-melting temperatures or become active upon exposure to another energy source, e.g. UV light or electron beam radiation, are suitable.

Optionally, the silicone pressure sensitive adhesive may include fillers, such as extending or reinforcing fillers.

The ester used for making the silicone pressure sensitive adhesive disclosed in U.S. Patent No. 2,857,356 to Goodwin as described *supra* has the general formula: 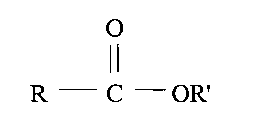 wherein R is a monovalent hydrocarbon radical having from 2 to 32 carbon atoms and R' is a monovalent hydrocarbon radical having from 1 to 14 carbon atoms. Preferably, R has from 10 to 19 carbon atoms and R' has from 1 to 3 carbon atoms. R and R' are selected independently, so that they may be the same or different.

Preferably, the esters for the hot-melt silicone pressure sensitive adhesives are not flammable which affords a safer procedure during application of the silicone pressure sensitive adhesive compositions at elevated temperatures. Flammable materials, as the term is used herein, are those materials, which are flammable according to the definition provided in United States Code of Federal Regulations, Title 49, Part 173, Section 115 (49 CFR 173.115). Briefly restated, a flammable liquid means any liquid having a flash point below 100°F (37.8°C), where flash point means the minimum temperature at which a liquid gives off vapour within a test vessel in sufficient concentration to form an ignitable mixture with air near the surface of the liquid. The CFR provides proper testing conditions for measuring flash point. If flammable esters are used, the coating operation could be done in an inert atmosphere (e.g. nitrogen gas), devoid of oxygen gas to avoid fire hazards.

The ester employed must not boil at the processing temperatures. Typically, temperatures above about 100°C produce suitable working viscosities with the hot melt silicone pressure sensitive adhesives, therefore, esters having boiling points above 100°C are preferred. The esters may be solid or liquid. Even though solid esters may be used, they must be at least somewhat soluble in the silicone pressure sensitive adhesive at the coating temperature.

Examples of suitable esters include 1-phenylethylpropionate, linolec acid ethyl ester, dodecyl acetate, ethyl triacontanoate, octyl acetate, methyl caproate, methyl decanoate, isobutyl acetate, methyl docosanoate, methyl heptadeconate, isopropylpalmitate, isopropylmyristate, lauric acid methyl ester and mixtures thereof.

The esters may be employed in amounts of about 1% to 10% by weight based on the total weight of the silicone resin and the silicone fluid. Generally, if the ester is a fluid at room temperature, especially when the pressure-sensitive adhesive is not to be cured, it is preferred that the maximum limit of the ester be about 7%, because at higher amounts, the ester may make the hot-melt silicone pressure sensitive adhesive too flowable at room temperature which is undesirable for most applications. Usually, the solid esters are preferred when it is desired to use greater than about 7% by weight ester in the hot melt silicone pressure sensitive adhesive.

The silicone pressure sensitive adhesives may be made by mixing the ingredients in any order. Reaction or treatment of the ingredients, e.g. condensing according to the procedure disclosed in CA 711,756 or chemically treating according to US 4,591,622 and US 4,584,355 may require completion prior to the addition of the ester.

The ester allows the hot melt silicone pressure sensitive adhesive to decrease in viscosity with elevated temperatures to a suitable viscosity for coating a substrate without the use of solvents that must be removed. Suitable viscosities for hot-melting processing are about 20,000-30,000 cp (centipoise) and, more typically, 30,000 - 40,000 cp. Typically, heating the hot-melt silicone pressure sensitive adhesives of this invention to temperatures of about 100°C or more (more typically above 150°C) result in suitable viscosities less than 40,000 cp. These coatable temperatures are low enough so that decomposition of the composition does not occur. Lower temperatures may result in coatable viscosities depending on the coating equipment used, the desired end product and the type and amount of ester used. For example, the thicker the layer of pressure sensitive adhesive desired, the higher the coating viscosity can be.

### The topsheet

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and non woven materials; polymeric materials such as apertured formed thermoplastic films, apertured plastic films, and hydroformed thermoplastic films; and thermoplastic scrims. Suitable woven and non-woven materials can be comprised of natural fibres (e.g., wood or cotton fibres), synthetic fibres (e.g., polymeric fibres such as polyester, polypropylene, or polyethylene fibres) or from a combination of natural and synthetic fibres or bi-/multi-component fibres.

Preferred topsheets for use in the present invention are selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the body remains dry; thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred micro apertured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the body surface of the topsheet can be made hydrophilic by treating it with a surfactant such as is described in U.S. 4,950,254.

Another alternative are so called hybrid topsheets, which incorporate fibrous and film like structures particularly useful embodiments of such hybrid topsheets are disclosed in PCT publications WO 93/09744; WO 93/11725 or WO 93/11726.

The topsheet typically extends across the whole of the absorbent structure and outside the area coextensive with the absorbent structure. The topsheet can extend and form part or all of the preferred side flaps, side wrapping elements or wings.

When referring to the topsheet a multi layer structure or a monolayer structure is contemplated. The hybrid topsheet mentioned above is such a multi layer design but other multi layer topsheets such as primary and secondary topsheet designs are also considered.

### Absorbent core

According to the present invention the absorbent cores suitable for use herein may be selected from any of the absorbent cores or core system known in the art. As used herein the term absorbent core refers to any material or multiple material layers whose primary function is to absorb, store and distribute fluid.

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

### a) Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers.

### b) Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared from polymerisable, unsaturated, acid-containing monomers, which are well known in the art.

Suitable carriers include materials, which are conventionally utilised in absorbent structures such as natural, modified or synthetic fibres, particularly modified or non-modified cellulose fibres, in the form of fluff and/or tissues. Suitable carriers can be used together with the absorbent gelling material; however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibres such as the stiffened cellulose fibres can also be used. Synthetic fibres can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibres, tricomponent fibres, mixtures thereof and the like. Preferably, the fibre surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c) Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibres, sheets or strips, this fibrous "dusting" layer need not be included. However, this "dusting" layer provides some additional fluid-handling capabilities such as rapid wicking of fluid along the length of the pad.

### d) Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component, which can be included in the absorbent core according to the invention and preferably is provided close to or as part off the primary or secondary fluid distribution layer are odour control agents.

### Backsheet

The backsheet primarily prevents the exudates absorbed and contained in the absorbent structure from wetting articles that contact the absorbent product such as underpants, pants, pyjamas and garments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

According to the present invention the backsheet of the absorbent article is preferably breathable such that it is moisture vapour permeable and thus comprises at least one gas permeable layer. Suitable gas permeable layers include 2 dimensional, planar micro and macro-porous films, macroscopically expanded films, formed apertured films and monolithic films. According to the present invention the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable 2 dimensional planar layers of the backsheet may be made of any material known in the art, but are preferably manufactured from commonly available polymeric materials. Suitable materials are for example Gortex™ or Sympatex™ type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EP 293,482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3,929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured performed films are uni directional such that they have at least substantially, if not complete one directional fluid transport towards the core.

Suitable macroscopically expanded films for use herein include films as described in for example in US 4,637,819 and US 4,591,523.

Suitable monolithic films include Hytrel™, available from DuPont Corporation, USA, and other such materials as described in Index 93 Congress, Session 7A "Adding value to Nonwovens", J-C.

Cardinal and Y. Trouilhet, DuPont de Nemours international S.A, Switzerland such as Pebax™, available from Elf Atochem (France) and Estane™ available from B.F. Goodrich (Belgium).

Particularly preferred backsheets for the present invention comprise at least two layers comprising at least one layer selected from the above, such as microporous and apertured formed films and an additional layer which may also be selected from the above listed backsheets or may be a fibrous woven or nonwoven. The most preferred breathable backsheet component comprises a microporous film and an apertured formed film or a microporous and a hydrophobic woven or nonwoven material.

The adhesive-coated surfaces are typically provided with protective covers, which are removed prior to use. Prior to use of the absorbent article the areas being coated with PFA are typically protected from contamination and from adhering to another surface, where this is not desired, by a protective cover means such as a silicone coated release paper, a plastic film or any other easily removable cover. The protective cover means can be provided as a single piece or in a multitude of pieces, e.g. to cover the individual adhesive areas. It also can perform other functions such as provide individualised packaging for the article or provide a disposal function. Any commercially available release paper or film may be used. Suitable examples include BL 30 MG-A SILOX EI/O, BL 30 MG-A SILOX 4 P/O available from Akrosil Corporation, and M&W films available from Gronau in Germany, under the code X-5432.

The PFA may be applied to the garment-facing surface of the backsheet or the wings using any one of methods well known in the art for this purpose such as slot coating, spraying and roll printing. With the development of adhesive printing as described for example in EP 745,432, EP 745,433, and EP 745,368 it has now also become possible to provide such panty-fastening adhesive in any desired shape and hence these methods are particularly preferred in the present invention. Preferably, the panty fastening adhesive is applied in intermittent patterns such as for example micro-sized intermittent dots, intermittent strips, lines or grids or other designed shapes such as circles. It is also within the scope of the present invention to apply the PFA in a completely random pattern, for example as achieved by spraying.

### Examples

In the following we report two examples of adhesives matching the rheology criteria of the present invention and having good adhesion performance on both microfibre and cotton. As reference for comparison we embody two adhesives (HL1461E ex H.B. Fuller, DMO110 ex National Starch), currently used in sanitary napkins on the market, which have a good performance on cotton but unacceptable performance on microfibre material.

| Adhesive | **HL1461E** | **DM0110** | **Bio PSA 7-4560** | **MF55** |
|---|---|---|---|---|
| tanδ at 0.01 Hz and 25°C | 0.06 | 0.13 | 1.5 | 0.48 |
| G' at 0.01Hz and 25°C, Pa | 26400 | 9000 | 1500 | 7100 |
| tanδ vs. frequency at 25°C | See fig. 5 | See fig. 5 | See fig. 5 | See fig. 5 |
| G' vs. frequency at 25°C | See Fig. 6 | See Fig. 6 | See Fig. 6 | See Fig. 6 |
| Peel on cotton, N/5cm | 2.4 | 2.3 | 4.50 | 1.96 |
| Peel on microfibre, N/5cm | 0.2 | 0.25 | 3.55 | 0.67 |

### For Bio PSA 7-4560 from DOW Corning

In this example the adhesive is coated between a plastic Film and release paper as described below:

Coating of the PFA onto the outer surface of the backsheet film was facilitated by first applying the PFA onto a release paper, which is available from Akrosil under the code BL XXG NL-MGA SILOX D3H/0 and having a basis weight of 40 g/m², at an application temperature of 200°C. After this coating step the PFA-coated release paper was transferred to the outer surface of the backsheet film by pressing the PFA-coated surface of the release paper onto said outer surface at a pressure of 2 bar. Thereby, the PFA was transferred from the release paper to the backsheet film and the release paper was removed afterwards. The PFA was Bio PSA 7-4560 available from DOW Corning and was applied with a full coating application 50 mm wide at a basis weight of 20 g/m². The film was a PE film 25 g/m² from Britton Taco available under the code ST-012-White. The PFA surface coverage was about 89% of the coated surface.

### For MF55 ex Savare:

This example is a panty liner made as follows:

The topsheet is a SBPP Nonwoven 21 g/m2 from BBA Fiberweb (France) under the code SPUN-S BLANC ITEM 111 DIMSET 021HI. The core is an Airlaid 100 g/m2 from Concert GmbH under the code GH100.1008. The backsheet is a SBPP nonwoven 23 g/m2 from BBA Neuberger available under the code BASE F1 023 01 001. The PFAs were applied as a full coating application (50 mm wide) done directly on the outer surface of the nonwoven backsheet with a PFA basis weight of 17 g/m2. The manner of applying the PFAs and the release paper used therefore was the same as for the Bio PSA 7-4560 of the previous example, while the application temperature was adapted to 155°C. The PFA surface coverage was about 45% of the coated surface.

### For the other two adhesives (DM0110, HL1461E):

These two examples were produced by the same procedure and materials as for Bio PSA 7-4560 from DOW Corning. Only the PFA application temperature was adapted to the specific adhesive, i.e. 155°C for HL1461E and 190°C for DM0110.
Additionally HL1461 has also been applied also on non-woven backsheet with the same procedure and materials as described for MF-55. The so-prepared example has provided 1.84N/5cm on cotton and 0.34N/5cm on microfibre.

### Peel force test method

### 1. Peel force on standard cotton material

An article of the present invention or part thereof (hereinafter sample) comprising on its garment-facing surface the PFA (the sample and PFA being at room temperature), is placed on a rigid support with the surface with the PFA facing upward, away from the support. Then a plate having an opening, which hereinafter is called "measurement window", is placed on top of the sample's surface, which comprises the PFA. The sample dimensions are to be chosen such that the sample at least fits the measurement window having dimension of 54 mm (width) x 126 mm (length). The sample is to be placed relative to the measurement window of the plate such that the measurement window is completely filled by the sample. In a typical execution of this test the sample is placed such that its midpoint (intersect of longitudinal and lateral centre lines) is congruent with the midpoint of the measurement window and that the longitudinal centerlines of the sample and the measurement window are parallel. The sample is fixed to the support by grips in a tight and wrinkle-free manner. Then a piece of cotton (100%), known as Weave Style no. 429W, available from Loeffler, Sitter Technic GmbH, Nettersheim, Germany, is placed on top of the surface with the PFA, which is exposed through the measurement window, such that one end of the cotton piece extends about 25 mm from the end of the measurement window with the PFA. The mwasurement window must be completely covered by the cotton piece. Then, a weight is placed on the thus formed sample-cotton combination for 30 seconds, such that the whole combination is covered and a weight of 26-27 g/cm² is applied, to ensure that the combination is pressed in a gentle and even manner.

Then, Zwick tensile tester (available from Zwick GmbH) is used to measure the peel force required to remove the cotton piece from the sample. Hereto, the support carrying the sample covered by the cotton piece is placed in the lower clamp of the tensile tester and the tail end of the cotton piece (the one opposite to the free 25 mm specified above) is placed in the upper clamp of the tensile tester. The Zwick tensile tester is set on a speed of 102 cm/min. Typically, the clamps are 250 mm spaced apart. It is obvious that any suitable constant rate of elongation tensile tester can be used.

Then, within 1 minute after removal of the compression weight, the tensile tester is started. The cotton piece is peeled off from the sample in a direction, which is parallel to the longitudinal dimension of the measurement window. During the peeling procedure the peel force required to peel off the cotton piece along the displacement of the upper clamp, which moved in an angle of 180° with the sample, is measured. The peel force is calculated as the average of the force peaks over a 13 cm path. The first 2.5 cm and last 3.75 cm of the measurement are not taken into account by the calculation of the peel force, to avoid influences of acceleration and deceleration.

The above test is for example done on a sample of the shape and size of a regular Always Alldays pantiliner, using a support plate with a measurement window of 54 mm (width) x 126 mm (length) and a weight of 2.1 kg with area dimensions 54 mm x 140 mm. The method can be easily adjusted by the skilled person for different sample sizes.

### 2. Peel force on standard microfibre material

This method is a variation of the method above for PFA peel strength measurement on cotton. Such variation has been designed to measure PFA peel strength from microfibre swatches instead of rigid cotton swatches. In fact when using the microfibre swatch, it will be stretched along the test, such stretching increasing a lot the variability of the method above for cotton material. Therefore it has been designed a specific sample preparation for the microfibre material with a tape, in order to make rigid the microfibre material, like rigid cotton swatch. Once the microfibre swatch has been prepared as described below, the test will be performed using the method above for cotton by just replacing the cotton swatch with the blocked microfibre swatch.

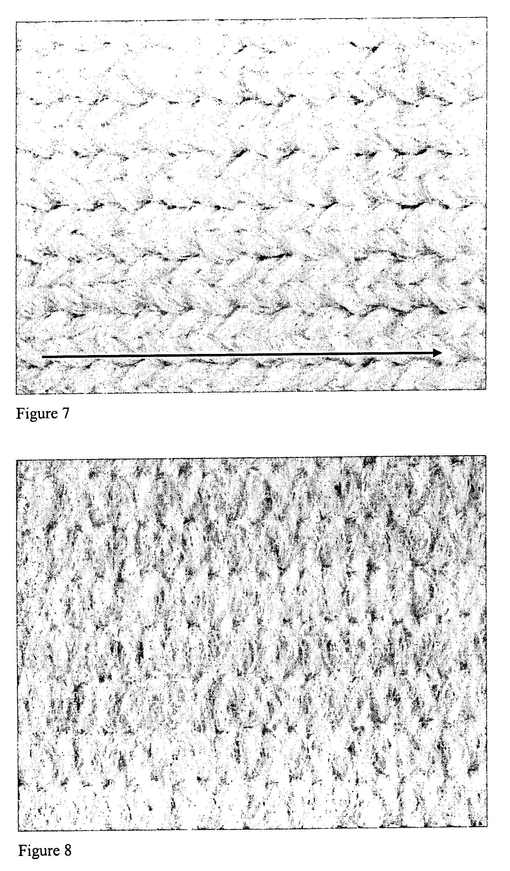

The extra tools, required for the microfibre material, are:
- Tape: P42, 70 mm width. Available from H-Old s.p.a. - 20010 Bareggio - MI - Italy - Via Monte Nero, 35, Tel. +39 0290360612 - Fax +39 0290362186
- Roller Weight: Steel cylinder having a weight of 1,14 kg and 6.5 cm wide.
- Microfibre swatches: 95% nylon, 5% elastan, color white, available from Maglificio Brugnoli Giovanni Sp.A. in Busto Arsizio (Va) Italy, under the code Zaffiro B/Fast ZAFF60TN, having a thickness of 0,7 mm, a basis weight of 160 g/m², dimensions of 457mm×76mm, oriented along the jersey pattern direction of the fabric (indicated with the black arrow in the Figure 7). Figure 7 is showing the tape side of the microfibre material, i.e. the side of the microfibre material onto which the tape is attached to block the microfibre swatch: as clear from the picture, such side is the textured one showing the fish bone like pattern of the jersey knitting). The PFA side (to be attached to the PFA) is the smoothest one (see Figure 8).

The blocked microfibre swatch will be prepared as follows:
- Take a microfibre swatch and hold it so that the PFA side is on the bottom
- Lay down the microfibre swatch onto a table.
- Lay down the tape onto the microfibre swatch, in order to cover the swatch. The tape piece must be as long as the microfibre swatch.
- Move the roller weight slowly twice at constant speed over the tape in the length direction, both times in the same sense. This will allow the tape to stick to the microfibre swatch. During the rolling, avoid additional pressure - only the weight of the roller must be applied.

The roller is only used to combine the P42 tape to the microfibre fabric.

In both test methods described hereinbefore the mean value of 10 measurements under identical conditions was used to obtain one date point.

All documents cited in the Detailed Description of the Invention are, in relevant part, incorporated by reference; the citation of any document is not to be construed as an admission that it is prior art with respect to the present invention.

While particular embodiments of the present invention have been illustrated and described, it would be obvious to those skilled in the art that various other changes and modifications can be made without departing from the spirit and scope of the invention. In particular it is obvious to the person skilled in the art that the present invention applies to microfibre materials with inherent hydrophobicity as well as to other panty materials, which were subjected to a hydrophobic treatment. It is therefore intended to cover in the appended claims all such changes and modifications that are within the scope of this invention.

## Claims

1. A disposable absorbent article for personal hygiene, said article having a wearer-facing surface and a garment-facing surface, said garment-facing surface comprising an adhesive for attachment of said article to the garment of a wearer,
**characterized in that** said adhesive has an elastic modulus G' at 0.01 Hz and 25°C of from 1,000 to 10,000 Pa and a loss tangent tanδ at 0.01 Hz and 25°C of from 0.3 to 2.

2. The article of claim 1, **characterized in that** said adhesive has an elastic modulus G' at 0.01 Hz and 25°C of from 1,500 to 8,000 Pa.

3. The article of any of the previous claims, **characterized in that** said adhesive has a loss tangent tanδ at 0.01 Hz and 25°C of from 0.4 to 1.6.

4. The article of any of the previous claims, **characterized in that** said adhesive has a tanδ residing inside a quadrangle ABCD wherein said quadrangle ABCD is defined by graphically plotting frequency in Hz versus tanδ referenced to 25°C of said adhesive, said quadrangle ABCD having as points A and D a tanδ of 0.3 and 2, respectively, at a frequency of 0.01 Hz and points B and C at a tanδ of 0.8 and 4, respectively, at a frequency of 10 Hz.

5. The article of any of the previous claims, **characterized in that** said adhesive has a tanδ residing inside a quadrangle ABCD wherein said quadrangle ABCD is defined by graphically plotting frequency in Hz versus tanδ referenced to 25°C of said adhesive, said quadrangle ABCD having as points A and D a tanδ of 0.4 and 1.6, respectively, at a frequency of 0.01 Hz and points B and C at a tanδ of 1 and 3, respectively, at a frequency of 10 Hz.

6. The article of any of the previous claims, **characterized in that** said adhesive has a G' residing inside a quadrangle ABCD wherein said quadrangle ABCD is defined by graphically plotting frequency in Hz versus tanδ referenced to 25°C of said adhesive, said quadrangle ABCD having as points A and D a G' of 1,000 and 10,000, respectively, at a frequency of 0.01 Hz and points B and C at a G' of 20,000 and 100,000, respectively, at a frequency of 10 Hz.

7. The article of any of the previous claims, **characterized in that** said adhesive has a G' residing inside a quadrangle ABCD wherein said quadrangle ABCD is defined by graphically plotting frequency in Hz versus tanδ referenced to 25°C of said adhesive, said quadrangle ABCD having as points A and D a G' of 1,500 and 8,000, respectively, at a frequency of 0.01 Hz and points B and C at a G' of 30,000 and 80,000, respectively, at a frequency of 10 Hz.

8. The article of any of the preceding claims, **characterized in that** said article is a sanitary napkin, a panty liner, a diaper, an underarm sweat pad, a hatband or an incontinence protection device.
